Europäisches Patentamt

European Patent Office

Office européen des brevets

(19)

(11) **EP 1 025 075 B1**

(12) **EUROPÄISCHE PATENTSCHRIFT**

(45) Veröffentlichungstag und Bekanntmachung des Hinweises auf die Patenterteilung:
**19.03.2003 Patentblatt 2003/12**

(21) Anmeldenummer: **98955421.7**

(22) Anmeldetag: **09.10.1998**

(51) Int Cl.[7]: **C07C 51/215**, C07C 51/25, B01J 23/652

(86) Internationale Anmeldenummer:
**PCT/EP98/06414**

(87) Internationale Veröffentlichungsnummer:
**WO 99/020592 (29.04.1999 Gazette 1999/17)**

(54) **VERFAHREN ZUR SELEKTIVEN HERSTELLUNG VON ESSIGSÄURE DURCH KATALYTISCHE OXIDATION VON ETHAN**

METHOD FOR SELECTIVELY PRODUCING ACETIC ACID THROUGH THE CATALYTIC OXIDATION OF ETHANE

PROCEDE DE PREPARATION SELECTIVE D'ACIDE ACETIQUE PAR OXYDATION CATALYTIQUE D'ETHANE

(84) Benannte Vertragsstaaten:
**AT BE CH DE ES FR GB IT LI NL SE**

(30) Priorität: **17.10.1997 DE 19745902**

(43) Veröffentlichungstag der Anmeldung:
**09.08.2000 Patentblatt 2000/32**

(73) Patentinhaber: **Celanese Chemicals Europe GmbH**
**60439 Frankfurt am Main (DE)**

(72) Erfinder:
• **BORCHERT, Holger**
  **D-67278 Bockenheim a.d. Weinstrasse (DE)**
• **DINGERDISSEN, Uwe**
  **D-64342 Seeheim-Jugenheim (DE)**

(56) Entgegenhaltungen:
**EP-A- 0 294 845**          **EP-A- 0 620 205**
**WO-A-98/05619**          **US-A- 4 499 301**

## Beschreibung

Die vorliegende Erfindung betrifft ein Verfahren zur selektiven Herstellung von Essigsäure durch katalytische Gasphasenoxidation von Ethan und /oder Ethylen in Gegenwart eines Palladium enthaltenden Katalysators.

Die oxidative Dehydrierung von Ethan zu Ethylen in der Gasphase, bei Temperaturen > 500°C ist beispielsweise aus US-A-4,250,346, US-A-4,524,236 und US-A-4,568,790 bekannt.

So beschreibt die US-A-4,250,346 die Verwendung einer Katalysatorzusammensetzung, die die Elemente Molybdän, X und Y im Verhältnis a:b:c enthält zur Umwandlung von Ethan in Ethylen, worin

X gleich Cr, Mn, Nb, Ta, Ti, V, und/oder W ist und Y gleich Bi, Ce, Co, Cu, Fe, K, Mg, Ni, P, Pb, Sb, Si, Sn, Tl und/oder U ist und a gleich 1, b gleich 0,05 bis 1 und c gleich 0 bis 2 ist. Der Gesamtwert von c für Co, Ni und/oder Fe muß dabei weniger als 0,5 betragen.

Die Reaktion wird vorzugsweise in Anwesenheit von zugefügtem Wasser durchgeführt. Die offenbarten Katalysatoren können ebenfalls zur Oxidation von Ethan zu Essigsäure verwendet werden, wobei die Effizienz der Umwandlung zu Essigsäure bei ca. 18 %, bei einer Ethan-Umwandlung von 7,5%, liegt.

Die vorstehend genannten Schriften beschäftigen sich hauptsächlich mit der Herstellung von Ethylen, weniger mit der gezielten Herstellung von Essigsäure.

Dagegen beschreibt die EP-B-0 294 845 ein Verfahren zur selektiven Herstellung von Essigsäure aus Ethan, Ethylen oder Gemischen davon mit Sauerstoff in Gegenwart eines Katalysatorgemisches, welches mindestens A.) einen calcinierten Katalysator der Formel $Mo_xV_y$ oder $Mo_xV_yZ_y$, worin Z eines oder mehrere der Metalle Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co und Ni sein kann, und x gleich 0,5 bis 0,9 ist. y gleich 0,1 bis 0,4 ist; und z gleich 0,001 bis 1 ist und

B.) einen Ethylenhydratationskatalysator und/oder Ethylenoxidationskatalysator enthält. Bei der zweiten Katalysatorkomponente B handelt es sich insbesondere um einen Molekularsiebkatalysator oder einen Palladium enthaltenden Oxidationskatalysator.

Bei der Verwendung des beschriebenen Katalysatorgemisches und Einspeisung eines Gasgemisches bestehend aus Ethan, Sauerstoff, Stickstoff und Wasserdampf durch den Katalysator enthaltenden Reaktor beträgt die maximale Selektivität 27% bei einem Ethanumsatz von 7%. Die hohen Umsatzraten von Ethan werden gemäß der EP 0 294 845 nur mit dem beschriebenen Katalysatorgemisch, nicht jedoch mit einem einzigen, die Komponenten A und B enthaltenden Katalysator erreicht.

Aus der EP-A-0 620 205 ist ein Verefahren zur Herstellung von Essigsäure bekannt.

Ein weiteres Verfahren zur Herstellung eines Produktes, das Ethylen und/oder Essigsäure enthält wird in EP-B-0 407 091 beschrieben. Hierbei werden Ethan und/oder Ethylen und ein molekularen Sauerstoff enthaltendes Gas bei erhöhter Temperatur mit einer Katalysatorzusammensetzung, die die Elemente A, X und Y enthält, in Kontakt gebracht. A ist hierbei $MO_dRe_eW_f$, X ist Cr, Mn, Nb, Ta, Ti, V und/oder W und Y ist Bi, Ce, Co, Cu, Fe, K, Mg, Ni, P, Pb, Sb, Si, Sn, Tl und/oder U. Die maximalen Selektivitäten, die bei Verwendung des beschriebenen Katalysators bei der Oxidation von Ethan zu Essigsäure erzielt werden konnten, betragen 78%. Als weitere Nebenprodukte werden Kohlendioxid, Kohlenmonoxid und Ethylen gebildet.

In der deutschen nicht-vorveröffentlichten Patentanmeldung P 19630832.1 wird ein Verfahren zur selektiven Herstellung von Essigsäure aus einer gasförmigen Einspeisung von Ethan, Ethylen oder Gemischen davon sowie Sauerstoff bei erhöhter Temperatur beschrieben. Die Einspeisung wird dabei mit einem Katalysator zusammengebracht, der die Elemente Mo, Pd, X und Y in Kombination mit Sauerstoff enthält.

X steht dabei für eines oder mehrere der Elemente ausgewählt aus der Gruppe Cr, Mn, Nb, Ta, Ti, V, Te und W und Y steht für eines oder mehrere der Elemente ausgewählt aus der Gruppe B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl und U;

Die Grammatomverhältnisse für die entsprechenden Elemente werden dabei wie folgt angegeben: a(Mo) = 1; b (Pd) >0; c(X) >0; und d(Y) = 0-2.

Die in oben genannter Anmeldung beschriebenen Katalysatoren zeigen eine maximale Raum-Zeit-Ausbeute von 149 kg/hm$^3$ bei einer Essigsäureselektivität von >60 Mol%. Raum-Zeit-Ausbeuten kennzeichnen die Menge der produzierten Essigsäure pro Zeit und Katalysatorvolumen. Höhere Raum-Zeit-Ausbeuten sind wünschenswert, da hierdurch die Größe der Reaktoren sowie die Menge des im Kreis geführten Gases verringert werden können.

Es besteht daher die Aufgabe ein Verfahren zur Verfügung zu stellen, das es erlaubt Ethan und/oder Ethylen in einfacher Weise, gezielt und mit hoher Selektivität und Raum-Zeit-Ausbeute unter möglichst milden Reaktionsbedingungen zu Essigsäure zu oxidieren.

Überraschend wurde nun gefunden, daß es möglich ist, bei Verwendung eines Katalysators, der die Elemente Molybdän und Palladium und eines oder mehrere Elemente aus der Gruppe Chrom, Mangan, Niob, Tantal, Titan, Vanadium, Tellur und/oder Wolfram enthält, Ethan und/oder Ethylen unter relativ milden Bedingungen, in einfacher Weise mit hoher Selektivität und hervorragenden Raum-Zeit-Ausbeuten zu Essigsäure zu oxidieren.

Die vorliegende Erfindung betrifft somit ein Verfahren zur selektiven Herstellung von Essigsäure aus einer gasförmigen Einspeisung aus Ethan, Ethylen oder Gemischen davon sowie Sauerstoff bei erhöhter Temperatur, wobei die gasförmige Einspeisung mit einem Katalysator bei einer Temperatur im Bereich von 200 bis 500°C und bei einem Druck im Reaktor im Bereich von 0,1 bis 5 MPa zusammengebracht wird, der die Elemente Mo, Pd, X und Y in den Grammatomverhältnissen a:b:c:d in Kombination mit Sauerstof enthält

$$Mo_a Pd_b X_c Y_d \qquad\qquad (I)$$

und die Symbole X und Y folgende Bedeutung haben:

X steht für eines oder mehrere Elemente ausgewählt aus der Gruppe Ta, V, Te und W, insbesondere V und W;
Y steht für eines oder mehrere Elemente ausgewählt aus der Gruppe Bi, Cu, Au, Ag, Li, K, Rb, Cs, Mg, Ca, Sr, Ba, Nb, Zr, Hf und Sb; die Indizes a, b, c und d stehen für die Grammatomverhältnisse der entsprechenden Elemente, wobei
a = 1,
b = 0,0001 bis 0,01,
c = 0,4 bis 1 und
d = 0,005 bis 1 ist und wobei der Katalysator mindestens die Elemente X gleich V und Y gleich Nb, Ca und Sb enthält und wobei die Raum-Zeit-Ausbeute bei der Oxidation zu Essigsäure bei >150 kg/hm$^3$ liegt. Insbesondere steht Y für Li.

Sofern X und Y für mehrere verschiedene Elemente stehen, können die Indices c und d ebenfalls mehrere unterschiedliche Werte annehmen.

Weiterhin betrifft die vorliegende Erfindung einen Katalysator zur selektiven Herstellung von Essigsäure enthaltend die Elemente Mo, Pd, X und Y in den Grammatomverhältnissen a:b:c:d in Kombination mit Sauerstoff.

Die Grammatomverhältnisse a:b:c:d liegen vorzugsweise in folgenden Bereichen:

a = 1;
b = 0,0001 bis 0,005;
c = 0,5 bis 0,8 und
d = 0,01 bis 0,3.

Palladiumgehalte im Katalysator, die über der angegebenen Obergrenze liegen führen bei dem erfindungsgemäßen Verfahren zu einer Begünstigung der Kohlendioxidbildung. Ferner werden höhere Palladiumgehalte allgemein auch deshalb vermieden, da sie den Katalysator unnötig verteuern. Dagegen wird bei Palladiumgehalten unterhalb des angegebenen Grenzwertes eine Bevorzugung der Ethylenbildung beobachtet.

Vorzugsweise enthält der erfindungsgemäß verwendete Katalysator außer den Elementen Molybdän und Palladium noch Vanadium, Niob, Antimon und Kalzium in Kombination mit Sauerstoff. Die Grammatomverhältnisse a:b:c$^1$:d$^1$:d$^2$:d$^3$ der Elemente Mo:Pd:V:Nb:Sb:Ca sind vorzugsweise wie folgt:

a (Mo)=1;
b (Pd)= 0,0001 bis 0,005, insbesondere 0,0001 bis 0,001;
c$^1$ (V)= 0,4 bis 1,0;
d$^1$ (Nb)= 0,01 bis 0,2;
d$^2$ (Sb)= 0,01 bis 0,3;
d$^3$(Ca)= 0,01 bis 0,3;

Beispiele für derartige im erfindungsgemäßen Verfahren bevorzugt eingesetzte Katalysatorzusammensetzungen sind:

$$Mo_{1,00} Pd_{0,0005} V_{0,45} Nb_{0,03} Sb_{0,01} Ca_{0,01}$$

$$Mo_{1,00} Pd_{0,0005} V_{0,45} Nb_{0,03} Sb_{0,01} Ca_{0,01} K_{0,015}$$

$$Mo_{1,00}Pd_{0,00075}V_{0,45}Nb_{0,03}Sb_{0,01}Ca_{0,01}$$

$$Mo_{1,00}Pd_{0,00075}V_{0,55}Nb_{0,03}Sb_{0,01}Ca_{0,01}$$

$$Mo_{1,00}Pd_{0,00075}V_{0,45}Nb_{0,06}Sb_{0,01}Ca_{0,01}$$

$$Mo_{1,00}Pd_{0,0008}V_{0,55}Nb_{0,06}Sb_{0,01}Ca_{0,01}$$

$$Mo_{1,00}Pd_{0,00085}V_{0,55}Nb_{0,06}Sb_{0,01}Ca_{0,01}$$

$$Mo_{1,00}Pd_{0,00075}V_{0,55}Nb_{0,09}Sb_{0,01}Ca_{0,01}$$

$$Mo_{1,00}Pd_{0,0008}V_{0,50}Nb_{0,15}Te_{0,01}Ca_{0,01}$$

$$Mo_{1,00}Pd_{0,00075}V_{0,50}Nb_{0,09}W_{0,01}Pd_{0,0003}$$

Die erfindungsgemäß verwendeten Katalysatoren können nach den herkömmlichen Verfahren hergestellt werden. Hierzu geht man von einer Aufschlämmung, insbesondere einer wässrigen Lösung, die die einzelnen Ausgangskomponenten der Elemente entsprechend ihrer Anteile enthält, aus.

Die Ausgangsmaterialien der Einzelkomponente zur Herstellung des erfindungsgemäßen Katalysators sind neben den Oxiden vorzugsweise in Wasser lösliche Substanzen wie Ammoniumsalze, Nitrate, Sulfate, Halogenide, Hydroxide und Salze organischer Säuren, die durch Erwärmung in die entsprechenden Oxide umgewandelt werden können. Zur Vermischung der Komponenten werden wäßrige Lösungen oder Suspensionen der Metallsalze hergestellt und vermischt.

Bei Molybdän empfiehlt es sich aufgrund der kommerziellen Verfügbarkeit als Ausgangsverbindungen die entsprechenden Molybdate, wie z.B. Ammoniummolybdat, einzusetzen.

Als Palladiumverbindungen kommen beispielsweise Palladium(II)-chlorid, Palladium(II)-sulfat, Palladium(II)-tetraminnitrat, Palladium(II)-nitrat sowie Palladium(II)-acetylacetonat in Frage.

Die erhaltene Reaktionsmischung wird dann 5 Minuten bis 5 Stunden bei 50 bis 100 °C gerührt. Anschließend wird das Wasser entfernt und der verbleibende Katalysator bei einer Temperatur von 50 bis 150°C, insbesondere 80 bis 120°C getrocknet.

Für den Fall, daß der erhaltene Katalysator anschließend noch einem Kalzinierungsprozeß unterworfen wird, empfiehlt es sich den getrockneten und pulverisierten Katalysator bei einer Temperatur im Bereich von 100°C bis 800°C, insbesondere 200 bis 500°C in Gegenwart von Stickstoff, Sauerstoff oder eines sauerstoffhaltigen Gases zu kalzinieren. Die Zeitdauer beträgt 2 bis 24 Stunden.

Der Katalysator kann ohne ein entsprechendes Trägermaterial eingesetzt werden oder mit einem solchen gemischt oder auf ein solches aufgebracht werden. Geeignet sind übliche Trägermaterialien, wie z.B. poröses Siliziumdioxid, geglühtes Siliziumdioxid, Kieselgur, Kieselgel, poröses oder nicht poröses Aluminiumoxid, Titandioxid, Zirkoniumdioxid, Thoriumdioxid, Lanthanoxid, Magnesiumoxid, Calciumoxid, Bariumoxid, Zinnoxid, Cerdioxid, Zinkoxid, Boroxid, Bornitrid, Borcarbid, Borphosphat, Zirkoniumphosphat, Aluminiumsilikat, Siliziumnitrid oder Siliziumcarbid aber auch Glas-, Kohlefaser-, Metalloxid- oder Metallnetze oder entsprechende Monolithe.

Bevorzugte Trägermaterialien haben eine Oberfläche von weniger als 100 m$^2$/g. Bevorzugte Trägermaterialien sind Siliziumdioxid und Aluminiumoxid mit geringer spezifischer Oberfläche. Der Katalysator kann nach der Formgebung als regelmäßig oder unregelmäßig geformter Trägerkörper oder aber in Pulverform als heterogener Oxidationskatalysator eingesetzt werden.

Die Reaktion kann in der Wirbelschicht oder in einem Festbettreaktor durchgeführt werden. Für den Einsatz in einer Wirbelschicht wird der Katalysator auf eine Korngröße im Bereich von 10 bis 200 μm gemahlen.

Die gasförmige Einspeisung enthält Ethan und/oder Ethylen, welche als reine Gase oder in Mischung mit einem oder mehreren anderen Gasen dem Reaktor zugeführt werden. Als solche zusätzlichen oder Trägergase kommen beispielsweise Stickstoff, Methan, Kohlenmonoxid, Kohlendioxid, Luft und/oder Wasserdampf in Frage. Das molekularen Sauerstoff enthaltende Gas kann Luft oder ein an molekularen Sauerstoff reicheres oder ärmeres Gas als Luft, z.B. Sauerstoff, sein. Der Anteil des Wasserdampfes kann im Bereich von 0 bis 50 Vol% liegen. Höhere Wasserdampfkonzentrationen würden die Aufarbeitung der anfallenden wässrigen Essigsäure aus verfahrenstechnischen Gründen unnötig verteuern. Das Verhältnis von Ethan/Ethylen zu Sauerstoff liegt günstiggerweise im Bereich zwischen 1:1 und 10:1, vorzugsweise 2:1 und 8:1. Höhere Sauerstoffgehalte sind bevorzugt, da der erreichbare Ethanumsatz und somit die Ausbeute an Essigsäure höher ist. Bevorzugt ist die Zugabe von Sauerstoff oder des molekularen Sauerstoff enthaltenen Gases in einem Konzentrationsbereich außerhalb der Explosionsgrenzen unter Reaktionsbedingungen, da hierdurch die Durchführung des Verfahrens vereinfacht wird. Allerdings ist es auch möglich das Ethan/Ethylen zu Sauerstoffverhältnis innerhalb der Explosionsgrenzen einzustellen.

Die Reaktion wird bei Temperaturen zwischen 200 und 500°C, bevorzugt 200 bis 400°C durchgeführt. Der Druck kann atmosphärisch oder superatmosphärisch sein, z.B. im Bereich zwischen 0,1 und 5,0 MPa, bevorzugt 0,1 bis 3,0 MPa.

Die Reaktion kann in einem Festbett- oder Wirbelschichtreaktor durchgeführt werden. Zweckmäßgerweise wird Ethan zunächst mit den inerten Gasen wie Stickstoff oder Wasserdampf gemischt, bevor Sauerstoff oder das molekularen Sauerstoff enthaltende Gas zugeführt wird. Die vermischten Gase werden bevorzugt in einer Vorheizzone auf die Reaktionstemperatur vorgeheizt, bevor das Gasgemisch mit dem Katalysator in Kontakt gebracht wird. Aus dem Reaktorabgas wird Essigsäure durch Kondensation abgetrennt. Die übrigen Gase werden an den Reaktoreingang zurückgeführt, wo Sauerstoff oder das molekularen Sauerstoff enthaltende Gas sowie Ethan und/oder Ethylen zudosiert wird.

Bei einem Vergleich der erfindungsgemäß eingesetzten Katalysatoren mit denen im Stand der Technik bekannten findet man, daß mit den vorliegenden Katalysatoren unter gleichen Reaktionsbedingungen (Reaktionseingangsgas, Druck, Temperatur) höhere Raum-Zeit-Ausbeuten und Essigsäureselektivitäten erreicht werden.

Bei Verwendung des erfindungsgemäß eingesetzten Katalysators liegt die Selektivität bei der Oxidation von Ethan und/oder Ethylen zu Essigsäure bei ≥70 Mol%, vorzugsweise ≥80 Mol%, insbesondere ≥90 Mol%, und die Raum-Zeit-Ausbeute bei >150 kg/hm$^3$, insbesondere >200 kg/hm$^3$ vorzugsweise >300 kg/hm$^3$, so daß mit dem erfindungsgemäßen Verfahren im Vergleich mit dem Stand der Technik eine Erhöhung der Essigsäureausbeuten, bei gleichzeitiger Verminderung des Anfalls von ungewünschten Nebenprodukten, auf einfache Weise erzielt werden kann.

Beispiele:

Die in den Beispielen aufgeführte Katalysatorzusammensetzung ist in relativen Atomverhältnissen angegeben.

Katalysatorpräparation:

Katalysator (I):

Ein Katalysator mit folgender Zusammensetzung wurde hergestellt:

$$Mo_{1,00}Pd_{0,00075}V_{0,45}Nb_{0,03}Sb_{0,01}Ca_{0,01}$$

Lösung 1:

40 g Ammoniummolybdat in 100 ml Wasser

Lösung 2:

12,0 g Ammoniummetavanadat in 200 ml Wasser

Lösung 3:

4,19 g Nioboxalat, 0,96 g Antimonoxalat, 0,68 g Calciumnitrat in 100 ml Wasser

Lösung 4:

0,039 g Palladiumacetat in 100 ml Acton.

Die wässrigen Lösungen 1 bis 3 werden separat bei 70°C für 15 Minuten gerührt. Dann wird die dritte zur zweiten hinzugegeben. Die vereinigten Mischungen werden bei 70°C für 15 Minuten gerührt bevor diese zur ersten gegeben werden. Hiernach gibt man Lösung 4 hinzu. Die erhaltene Mischung wird bei 70°C für 15 Minuten gerührt und anschließend auf ein Volumen von 400 ml Lösungen eingedamft. Das Gemisch wird sprühgetrocknet und in statischer Luft bei 120°C für 2 Stunden und 300°C für 5 Stunden kalziniert. Der Katalysator wird hiernach leicht gemörsert und zu Tabletten gepreßt. Diese werden über ein Sieb zerstoßen, um eine Siebfraktion zwischen 0,35 und 0,7 mm zu erhalten.

Katalysator (II):

Ein Katalysator mit folgender Zusammensetzung wurde hergestellt:

$$Mo_{1,00}Pd_{0,0005}V_{0,45}Nb_{0,03}Sb_{0,01}Ca_{0,01}$$

Lösung 1:

40 g Ammoniummolybdat in 100 ml Wasser

Lösung 2:

12,0 g Ammoniummetavanadat in 200 ml Wasser

Lösung 3:

4,19 g Nioboxalat, 0,96 g Antimonoxalat, 0,68 g Calciumnitrat in 100 ml Wasser

Lösung 4:

0,026 g Palladiumacetat in 100 ml Acton.

Die Herstellung des Katalysators erfolgte wie in Katalysatorbeispiel (I) beschrieben.

Katalysator (III):

Ein Katalysator mit folgender Zusammensetzung wurde hergestellt:

$$Mo_{1,00}Pd_{0,00085}V_{0,55}Nb_{0,06}Sb_{0,01}Ca_{0,01}$$

Lösung 1:40 g Ammoniummolybdat in 100 ml Wasser

Lösung 2:

14,7 g Ammoniummetavanadat in 200 ml Wasser

Lösung 3:

8,38 g Nioboxalat, 0,96 g Antimonoxalat, 0,68 g Calciumnitrat in 100 ml Wasser

Lösung 4:

0,044 g Palladiumacetat in 100 ml Aceton.

Die Herstellung des Katalysators erfolgte wie in Katalysatorbeispiel (1) beschrieben.

Katalysator (IV):

Ein Katalysator mit folgender Zusammensetzung wurde hergestellt:

$$M_{1,00}Pd_{0,00075}V_{0,55}Nb_{0,09}Sb_{0,01}Ca_{0,01}$$

Lösung 1:

40 g Ammoniummolybdat in 100 ml Wasser

Lösung 2:

14,7 g Ammoniummetavanadat in 200 ml Wasser

Lösung 3:

12,75 g Nioboxalat, 0,96 g Antimonoxalat, 0,68 g Calciumnitrat in 100 ml Wasser

Lösung 4:

0,039 g Palladiumacetat in 100 ml Acton.
Die Herstellung des Katalysators erfolgte wie in Katalysatorbeispiel (I) beschrieben.

Katalysator (V):

Ein Katalysator mit folgender Zusammensetzung wurde hergestellt:

$$Mo_{1,00}Pd_{0,00085}V_{0,55}Nb_{0,09}Sb_{0,01}Ca_{0,01}$$

Lösung 1:

40 g Ammoniummolybdat in 100 ml Wasser

Lösung 2:

14,7 g Ammoniummetavanadat in 200 ml Wasser

Lösung 3:

12,75 g Nioboxalat, 0,96 g Antimonoxalat, 0,68 g Calciumnitrat in 100 ml Wasser

Lösung 4:

0,044 g Palladiumacetat in 100 ml Acton.
Die Herstellung des Katalysators erfolgte wie in Katalysatorbeispiel (I) beschrieben.

Vergleichsbeispiel

Katalysator (VI)

Katalysatorbeispiel (I) aus der deutschen nicht-vorveröffentlichten Patentanmeldung P 19630832.1 mit der Zusammensetzung $Mo_{1,00}Pd_{0,0005}V_{0,25}Nb_{0,12}$
wurde hergestellt, um die höhere Raum-Zeit-Ausbeute der erfindungsgemäßen Katalysatoren zu zeigen.

Lösung 1:

61,75 g Ammoniummolybdat und 0,039 g Palladiumacetat in 200 ml Wasser

Lösung 2:

10,22 g Ammoniummetavanadat in 250 ml Wasser

Lösung 3:

27,51 g Nioboxalat 25 ml Wasser
Die Lösungen werden separat bei 90°C für 15 Minuten gerührt. Dann wird die dritte Lösung zur zweiten hinzugegeben. Die vereinigten Mischungen werden bei 90°C für 15 Minuten gerührt bevor die erste Lösung hinzugegeben wird. Die erhaltene Mischung wird bei 90°C für 15 Minuten gerührt. Anschließend wird das Gemisch sprühgetrocknet und in statischer Luft bei 120°C für 2 Stunden und 300°C für 5 Stunden kalziniert. Der Katalysator wird hiernach leicht gemörsert und zu Tabletten gepreßt. Diese werden über ein Sieb zerstoßen, um eine Siebfraktion zwischen 0,35 und 0,7 mm zu erhalten.

Methode zur Katalysatoraustestung:

5 oder 10 ml des Katalysators wurde in einen Stahlreaktor mit 10 mm Innendurchmesser geladen. Der Katalysator wurde unter einem Luftstrom auf 250°C aufgeheizt. Anschließend wurde der Druck mittels eines Vordruckreglers eingestellt. Das gewünschte Ethan:Sauerstoff:Stickstoff-Gemisch wurde mit Wasser in eine Verdampferzone eindosiert, wo Wasser verdampfte und mit den Gasen vermischt wurde. Die Reaktionstemperatur wurde mit einem Thermoelement in der Katalysatorschüttung gemessen. Das Reaktionsgas wurde on-line gaschromatographisch analysiert.
In den Beispielen sind die folgende Begriffe definiert als:

$$\text{Ethanumsatz (\%)} =$$

$$100x([CO]/2+[CO_2]/2+[C_2H_4]+[CH_3COOH])/([CO]/_2+[CO_2]/2+[C_2H_4]+[C_2H_6]+[CH_3COOH])$$

$$\text{Ethylenselektivität (\%)} =$$

$$100x([C_2H_4])/([CO]/_2+[CO_2]/2+[C_2H_4]+[CH_3COOH])$$

$$\text{Essigsäureselektivität (\%)} =$$

$$100x([CH_3COOH[)/([CO]/_2+[CO_2]/2+[C_2H_4]+CH_3COOH])$$

worin

[ ] = Konzentrationen in mol% und
$[C_2H_6]$ = Konzentration des nicht umgesetzten Ethans bedeutet.

Die Verweilzeit ist definiert als:

$$t \text{ (s)} = \text{Schüttvolumen des Katalysators (ml) / Volumenstrom des Gases durch den}$$

$$\text{Reaktor bezogen auf die Reaktionsbedingungen (ml/s)}$$

Reaktionsdurchführung:

Das Reaktoreingangsgas bestand aus 40 Vol% Ethan, 8 Vol% Sauerstoff, 32 Vol% Stickstoff und 20 Vol% Wasserdampf. Da die Raum-Zeit-Ausbeute vom Reaktionsdruck abhängig ist, wurden aus Gründen der Vergleichbarkeit alle Versuchsbeispiele bei 1,5 MPa durchgeführt. Reaktionsbedingungen und Ergebnisse sind in nachfolgender Tabelle zusammengefaßt.
Im Vergleich zu dem Vergleichskatalysator (VI) werden mit den Katalysatoren (I), bis (V) bei gleichen Temperaturen wesentlich höhere Selektivitäten und Raum-Zeit-Ausbeuten zu Essigsäure erreicht.

Tabelle 1:

| Bsp. | Katalysator | Temperatur (°C) | Druck (MPa) | Verweilzeit (s) | Ethanumsatz (%) | Essigsäure-selektivität (%) | Ethylen-selektivität (%) | Raum-Zeit-Ausbeute [kg/hm³] | CO+$CO_2$ Selektivität (%) |
|---|---|---|---|---|---|---|---|---|---|
| 1 | (I) | 300 | 1,5 | 14 | 15 | 63 | 25 | 184 | 11 |
| 2 | (II) | 300 | 1,5 | 14 | 15 | 69 | 19 | 193 | 12 |
| 3 | (III) | 300 | 1,5 | 10 | 10 | 86 | 2 | 247 | 12 |
| 4 | (IV) | 280 | 1,5 | 15 | 14 | 91 | 1 | 215 | 8 |
| 5 | (IV) | 300 | 1,5 | 10 | 14 | 89 | 3 | 355 | 8 |
| 6 | (IV) | 300 | 1,5 | 7 | 13 | 81 | 11 | 410 | 8 |
| 7 | (IV) | 310 | 1,5 | 7 | 15 | 85 | 3 | 470 | 12 |
| 8 | (V) | 300 | 1,5 | 10 | 15 | 77 | 14 | 325 | 9 |
| 9 | (VI) | 280 | 1,5 | 30 | 15 | 77 | 2 | 72 | 21 |

**EP 1 025 075 B1**

**Patentansprüche**

1. Verfahren zur selektiven Herstellung von Essigsäure aus einer gasförmigen Einspeisung von Ethan, Ethylen oder Gemischen davon sowie Sauerstoff bei erhöhter Temperatur, **dadurch gekennzeichnet, daß** die gasförmige Einspeisung mit einem Katalysator bei einer Temperatur im Bereich von 200 bis 500°C und bei einem Druck im Reaktor im Bereich von 0,1 bis 5 MPa zusammengebracht wird, der die Elemente Mo, Pd, X und Y in den Grammatomverhältnissen a:b:c:d in Kombination mit Sauerstoff enthält

$$Mo_aPd_bX_cY_d \qquad\qquad (I)$$

wobei die Symbole X und Y folgende Bedeutung haben

X steht für eines oder mehrere der Elemente ausgewählt aus der Gruppe Ta, V, Te und W;
Y steht für eines oder mehrere der Elemente ausgewählt aus der Gruppe Bi, Cu, Ag, Au, Li, K, Rb, Cs, Mg, Ca, Sr, Ba, Nb, Zr, Hf und Sb;
die Indizes a, b, c, d und x stehen für die Grammatomverhältnisse der entsprechenden Elemente, wobei
$a = 1$;
$b = 0,0001$ bis $0,01$;
$c = 0,4$ bis $1$;
und
$d = 0,005$ bis $1$ ist

und wobei der Katalysator mindestens die Elemente X gleich V und Y gleich Nb, Ca und Sb enthält und wobei die Raum-Zeit-Ausbeute bei der Oxidation zu Essigsäure bei $>150$ kg/hm$^3$ liegt.

2. Verfahren nach Anspruch 1, **dadurch gekennzeichnet, daß** X und/oder Y für mehrere Elemente stehen, wobei gegebenenfalls die Indizes c und d für verschiedene Elemente unterschiedliche Werte annehmen.

3. Verfahren nach Anspruch 1 oder 2, **dadurch gekennzeichnet, daß** b im Bereich von 0,0001 bis 0,001 liegt.

4. Verfahren nach mindestens einem der Ansprüche 1 bis 3, **dadurch gekennzeichnet, daß** dem Reaktor Ethan gemischt mit mindestens einem weiteren Gas zugeführt wird.

5. Verfahren nach Anspruch 4, **dadurch gekennzeichnet, daß** als weiteres Gas Stickstoff, Sauerstoff, Methan, Kohlenmonoxid, Kohlendioxid, Ethylen und/oder Wasserdampf zugeführt wird.

6. Verfahren nach mindestens einem der Ansprüche 1 bis 5, **dadurch gekennzeichnet, daß** der Katalysator mindestens eine der folgenden Zusammensetzungen in Kombination mit Sauerstoff enthält.

$$Mo_{1,00}Pd_{0,0005}V_{0,45}Nb_{0,03}Sb_{0,01}Ca_{0,01}$$

$$Mo_{1,00}Pd_{0,0005}V_{0,45}Nb_{0,03}Sb_{0,01}Ca_{0,01}K_{0,015}$$

$$Mo_{1,00}Pd_{0,00075}V_{0,45}Nb_{0,03}Sb_{0,01}Ca_{0,01}$$

$$Mo_{1,00}Pd_{0,00075}V_{0,55}Nb_{0,03}Sb_{0,01}Ca_{0,01}$$

$$Mo_{1,00}Pd_{0,00075}V_{0,45}Nb_{0,06}Sb_{0,01}Ca_{0,01}$$

$$Mo_{1,00}Pd_{0,0008}V_{0,55}Nb_{0,06}Sb_{0,01}Ca_{0,01}$$

$$Mo_{1,00}Pd_{0,00085}V_{0,55}Nb_{0,06}Sb_{0,01}Ca_{0,01}$$

$$Mo_{1,00}Pd_{0,00075}V_{0,55}Nb_{0,09}Sb_{0,01}Ca_{0,01}$$

**7.** Verfahren nach mindestens einem der Ansprüche 1 bis 6, **dadurch gekennzeichnet, daß** der Katalysator mit einem Trägermaterial gemischt oder auf einem Trägermaterial fixiert ist.

**8.** Verfahren nach mindestens einem der Ansprüche 1 bis 7, **dadurch gekennzeichnet, daß** die Selektivität der Oxidationsreaktion von Ethan und/oder Ethylen zu Essigsäure ≥70 Mol% liegt.

**Claims**

**1.** A process for the selective preparation of acetic acid from a gaseous feed comprising ethane, ethylene or mixtures thereof plus oxyen at elevated temperature, which comprises bringing the gaseous feed into contact at a temperature in the range of from 200 to 500°C and at a pressure in the range of from 0.1 to 5 MPa in the reactor with a catalyst comprising the elements Mo, Pd, X and Y in the gram atom ratios a:b:c:d in combination with oxygen

$$Mo_aPd_bX_cY_d \tag{I}$$

where the symbols X and Y have the following meanings:

X is one or more elements selected from the group consisting of Ta, V, Te and W;
Y is one or more elements selected from the group consisting of Bi, Cu, Ag, Au, Li, K, Rb, Cs, Mg, Ca, Sr, Ba, Nb, Zr, Hf and Sb;
the indices a, b, c, d and x are the gram atom ratios of the corresponding elements, where
a = 1;
b = 0.0001-0.01,
c = 0.4-1;
and
d = 0.005-1

and wherein the catalyst comprises at least the elements X = V and Y = Nb, Ca and Sb and wherein the space-time yield in the oxidation to acetic acid is > 150 kg/hm$^3$.

**2.** The process as claimed in claim 1, wherein X and/or Y are a plurality of elements and the indices c and d may assume different values for different elements.

**3.** The process as claimed in claim 1 or 2, wherein b is in the range from 0.0001 to 0.001.

**4.** The process as claimed in at least one of claims 1 to 3, wherein ethane mixed with at least one further gas is fed to the reactor.

**5.** The process as claimed in claim 4, wherein the further gas fed in is nitrogen, oxygen, methane, carbon monoxide, carbon dioxide, ethylene and/or water vapor.

**6.** The process as claimed in at least one of claims 1 to 5, wherein the catalyst comprises at least one of the following compositions in combination with oxygen:

$$Mo_{1.00}Pd_{0.0005}V_{0.45}Nb_{0.03}Sb_{0.01}Ca_{0.01}$$

$$Mo_{1.00}Pd_{0.0005}V_{0.45}Nb_{0.03}Sb_{0.01}Ca_{0.01}K_{0.015}$$

$$Mo_{1.00}Pd_{0.00075}V_{0.45}Nb_{0.03}Sb_{0.01}Ca_{0.01}$$

$$Mo_{1.00}Pd_{0.00075}V_{0.55}Nb_{0.03}Sb_{0.01}Ca_{0.01}$$

$$Mo_{1.00}Pd_{0.00075}V_{0.45}Nb_{0.06}Sb_{0.01}Ca_{0.01}$$

$$Mo_{1.00}Pd_{0.0008}V_{0.55}Nb_{0.06}Sb_{0.01}Ca_{0.01}$$

$$Mo_{1.00}Pd_{0.00085}V_{0.55}Nb_{0.06}Sb_{0.01}Ca_{0.01}$$

$$Mo_{1.00}Pd_{0.00075}V_{0.55}Nb_{0.09}Sb_{0.01}Ca_{0.01}$$

**7.** The process as claimed in at least one of claims 1 to 6, wherein the catalyst is mixed with a support material or fixed on a support material.

**8.** The process as claimed in at least one of claims 1 to 7, wherein the selectivity of the oxidation reaction of ethane and/or ethylene to acetic acid is $\geq$ 70 mol%.

**Revendications**

**1.** Procédé de préparation sélective d'acide acétique à partir d'un courant d'alimentation gazeux constitué d'éthane, d'éthylène ou de leurs mélanges et d'oxygène à haute température, **caractérisé en ce que** l'on met le courant d'alimentation gazeux en contact avec un catalyseur, à une température comprise entre 200 et 500°C et sous une pression dans le réacteur comprise entre 0,1 et 5 MPa, le catalyseur contenant les éléments Mo, Pd, X et Y dans les rapports en atomes-grammes a:b:c:d en combinaison avec de l'oxygène:

$$Mo_aPd_bX_cY_d \qquad (I)$$

les symboles X et Y ayant la signification suivante:

X représente un ou plusieurs éléments choisis dans le groupe constitué par Ta, V, Te et W;
Y représente un ou plusieurs éléments choisis dans le groupe constitué par Bi, Cu, Ag, Au, Li, K, Rb, Cs, Mg, Ca, Sr, Ba, Nb, Zr, Hf et Sb;
les indices a, b, c et d représentent les rapports en atomes-grammes des éléments correspondants, où
a = 1,
b = 0,0001 à 0,01,
c = 0,4 à 1 et
d = 0,005 à 1,

le catalyseur contenant au moins les éléments X = V et Y = Nb, Ca et Sb, et le rendement espace-temps pour l'oxydation en acide acétique étant supérieur à 150 kg/hm$^3$.

**2.** Procédé selon la revendication 1, **caractérisé en ce que** X et/ou Y représentent plusieurs éléments, et les indices c et d prennent éventuellement des valeurs différentes pour les différents éléments.

**3.** Procédé selon la revendication 1 ou 2, **caractérisé en ce que** b est compris entre 0,0001 et 0,001.

**4.** Procédé selon l'une au moins des revendications 1 à 3, **caractérisé en ce que** l'on introduit dans le réacteur de l'éthane mélangé avec au moins un autre gaz.

5. Procédé selon la revendication 4, **caractérisé en ce que** l'on introduit comme autre gaz de l'azote, de l'oxygène, du méthane, du monoxyde de carbone, du dioxyde de carbone, de l'éthylène et/ou de la vapeur d'eau.

6. Procédé selon l'une au moins des revendications 1 à 5, **caractérisé en ce que** le catalyseur contient au moins l'une des compositions suivantes en combinaison avec de l'oxygène:

$$Mo_{1,00}Pd_{0,0005}V_{0,45}Nb_{0,03}Sb_{0,01}Ca_{0,01}$$

$$Mo_{1,00}Pd_{0,0005}V_{0,45}Nb_{0,03}Sb_{0,01}Ca_{0,01}K_{0,015}$$

$$Mo_{1,00}Pd_{0,00075}V_{0,45}Nb_{0,03}Sb_{0,01}Ca_{0,01}$$

$$Mo_{1,00}Pd_{0,00075}V_{0,55}Nb_{0,03}Sb_{0,01}Ca_{0,01}$$

$$Mo_{1,00}Pd_{0,00075}V_{0,45}Nb_{0,06}Sb_{0,01}Ca_{0,01}$$

$$Mo_{1,00}Pd_{0,00085}V_{0,55}Nb_{0,06}Sb_{0,01}Ca_{0,01}$$

$$Mo_{1,00}Pd_{0,00085}V_{0,55}Nb_{0,06}Sb_{0,01}Ca_{0,01}$$

$$Mo_{1,00}Pd_{0,00075}V_{0,55}Nb_{0,09}Sb_{0,01}Ca_{0,01}$$

7. Procédé selon l'une au moins des revendications 1 à 6, **caractérisé en ce que** le catalyseur est mélangé avec un support ou fixé sur un support.

8. Procédé selon l'une au moins des revendications 1 à 7, **caractérisé en ce que** la sélectivité de la réaction d'oxydation de l'éthane et/ou de l'éthylène en acide acétique est d'au moins 70 % en mol.